# EUROPEAN PATENT SPECIFICATION

(11) **EP 4 214 488 B1**
(45) Date of publication and mention of the grant of the patent: **05.11.2025**
(21) Application number: 21794209.3
(22) Date of filing: 14.09.2021
(51) Int. Cl.: G01N 21/3563, G01N 21/359, G01N 21/47, G01N 33/02

(54) **MEASURING DEVICE FOR MEASURING FIRMNESS OF PRODUCTS, SUCH AS FRUIT AND VEGETABLES, SORTING SYSTEM PROVIDED THEREWITH AND METHOD THEREFOR**
MESSVORRICHTUNG ZUR MESSUNG DER FESTIGKEIT VON PRODUKTEN WIE OBST UND GEMÜSE, SORTIERSYSTEM DAMIT UND VERFAHREN DAFÜR
DISPOSITIF DE MESURE PERMETTANT LA MESURE DE LA FERMETÉ DE PRODUITS, TELS QUE DES FRUITS ET DES LÉGUMES, SYSTÈME DE TRI LE COMPRENANT ET PROCÉDÉ ASSOCIÉ

(30) Priority: 16.09.2020 NL 2026483
(43) Date of publication of application: 26.07.2023
(73) Proprietor: De Greef's Wagen-, Carrosserie- en Machinebouw B.V., 4196 JB Tricht (NL)
(72) Inventor: NIJLAND, Wilhelm Jan, 4196 JB Tricht (NL)
(74) Representative: Arnold & Siedsma
(86) International application number: PCT/NL2021/050556
(87) International publication number: WO 2022/060220

(56) References cited:
- US-A- 3 470 737
- US-A1- 2018 252 691

## Description

The present invention relates to a measuring device for measuring the firmness of products, particularly of fruit and vegetables. Products are particularly kiwis, mangoes, berries, apples, peaches, as well as tomatoes, peppers, potatoes and the like.

Measuring devices for performing a firmness measurement which are known in practice for instance make use of a penetrometer. Particularly measured hereby is the penetration resistance. In practice this is difficult to perform at high speed, and products are damaged thereby. Such a measurement is particularly unsuitable for a sorting system.

US 2018/252691 A1 discloses a measuring device for measuring firmness of products, such as fruit and vegetables, sorting system provided therewith and method therefor.

Other measuring devices known in practice make use of non-destructive measurements in order to counteract the risk of damage, and make use here of an acoustic signal, the reflection or transmission of which is measured, or wherein use is made of near-infrared spectrometry. Distorting effects, such as ambient light, have a relatively great deal of influence on the obtained measurement here. This causes an uncertainty in the obtained measurement result. It has also been found difficult to perform the measurements in reliable manner at high speed, particularly in the case of moving products.

In addition to the problem of conventional firmness measurements having been found to be insufficiently accurate in practical applications, there are further deviations in the accuracy as a result of variation in for instance dimensions and/or shape of the product. In addition, relatively "fresh" damage is insufficiently visible with conventional measurements, including spectrometry, because the internal decomposition process in the product with such damage is usually still getting started before such damage becomes detectable. For such products this results in wrongfully being assigned a good classification.

The present invention has for its object to provide a measuring device for measuring firmness of products such as fruit and vegetables, whereby the above stated problems are obviated or at least reduced.

The present invention provides for this purpose a measuring device according to claim 1 for measuring firmness of products, such as fruit and vegetables.

The measuring device according to the invention is able to measure the firmness of products. Such products are for instance kiwis, mangoes, avocados, berries, apples, peaches, tomatoes, peppers, potatoes and the like. Such a measuring device is for instance applied in a sorting system wherein products are sorted for further processing on the basis of the firmness measurement and/or other measurements.

According to the invention, the measuring device is provided with a signal device wherein one or more light sources are configured to transmit a number of measuring signals to the product while the product is being advanced in a transport direction. The number of measuring signals is in this case 1, 2, 3 or any other whole number. In the context of the invention light sources are also referred to as illuminating units. In the context of the invention transmitting a measuring signal to the product likewise comprises of irradiating the product with a light signal. The number of light sources can here be 1, 2, 3, 4, 10, 20 or any other suitable number of light sources. The light sources here transmit a suitable measuring signal which radiates onto the surface of the relevant product to be measured. The one or more light sources particularly irradiate a (beamed) light signal or light signals onto a part of the product surface. A part of the product surface therefore remains substantially non-irradiated. A (part of the) product surface is preferably irradiated substantially in the centre of the product.

By providing a camera device, preferably placed at an angle to the signal device, one or more camera images of the product can be recorded. The camera images comprise the irradiated product surface and the scatter surface area. The surface area of this irradiated product surface is preferably determined on the basis of the one or more camera images. By placing a camera at an angle to the light source or light sources a problem with reflection is obviated or at least reduced. The camera and light source can optionally be placed in line, i.e. at an angle of 0 degrees. The camera or camera device is also referred to as vision module.
When the measuring signal is transmitted to the product by the light source, the camera preferably records at least one image of the relevant product, on which said irradiated product surface is visible, also referred to as spot. The measuring signal will partially penetrate under the outer product surface and there disperse, i.e. scatter. This is visible to the camera as a scatter surface area which corresponds with the area of the product on which the (light) signal scattered under the product surface is visible, also referred to as scatter region. This obtained image, or these obtained images, are taken with the camera device while the product advances. The (directly) irradiated product surface is a part of the product surface, particularly a relatively small part. It is hereby possible to determine the scatter surface area of the light which scatters under the irradiated product surface and is visible at the non-irradiated (dark) product surface.

The measuring device preferably comprises a synchronizer configured to synchronize the camera device to the movement of the products with the transport device, also referred to as conveyor, in the transport direction. The synchronizer adapts the action of taking of one or more image recordings to the location of the product and preferably ensures that the camera device records an image of the product, and particularly of the specific surface areas, at a fixed distance. In a currently preferred embodiment an image recording is here taken the moment that the light signal of the light source hits the product substantially in the centre from the top, wherein the light signal is preferably oriented substantially vertically.

Because the light source and the camera device can transmit measuring signals and generate images at high speed, a firmness can be determined reliably even during movement of the product. The movement of the product is for instance carried out by a conveyor with diabolos or another suitable conveying means. The signal device can here be activated in pulse-wise or continuous manner, wherein the camera device takes one or more images at desired moments.

By connecting the detection module operatively to the signal device and camera device (also referred to as vision module), and preferably to the above stated synchronizer, it is possible to determine the firmness of the product on the basis of the irradiated product surface and the scattering. The detection module can be provided in integrated manner with a vision module or co-act therewith in order to process measurement results. The product surfaces and scatter surface areas irradiated with the signal device form a measure of the firmness of the product. It has been found that it is hereby possible to perform firmness measurements at high speed. Experiments show that speeds of five products per track per second, up to as much as 8, 10, 20 and even more than 30 products per track per second are possible. The detection module for instance determines the size of the relevant surface areas by comparing values of the images to thresholds in order to determine that there is an irradiated product surface, also referred to as spot, or a scatter surface area, also referred to as scatter region. These thresholds are preferably also determined in accordance with the type of light source, type of product, variety and the like.

In a currently preferred embodiment according to the invention the number of light sources comprises a number of lasers.

The use of a laser makes it possible, among other things, to transmit a coherent light beam as measuring signal. Such a beam has a minimal convergence or divergence of the signal. In a currently preferred embodiment use is made of a point laser, although a line laser is alternatively also possible. It has however been found that a point laser is easiest to use and produces the most reliable results.

It is alternatively or additionally also possible to transmit measuring signals to the product in a specific pattern. It is possible if desired to project a pattern, line or point onto the product surface with a normal light source, optionally via a lens system. The applied measuring signals are preferably adapted to the type of product.

It has been found possible with a laser to perform a reliable measurement at high speed. It has here even been found possible also to detect "fresh" damage. This is probably because the measurement of the firmness does not depend on the (bio)chemical reaction/composition which changes as a result of the occurring damage. According to the invention, the firmness measurement therefore experiences no delay in detecting such "fresh" damage. The firmness measurements obtained with a measuring device according to the invention are therefore further quickly available and reliable. It will be apparent that, as an alternative to or in addition to the use of a laser, it is also possible to apply a different light source, for instance with infrared. It is likewise possible if desired to use additional detectors, including (near) infrared sensors, ultrasonic sensors or other suitable sensors. It is also possible here to apply combinations of these sensors in order to increase the accuracy of the firmness measurement still further.

It is likewise possible according to the invention to combine the measuring device with a measuring system of a sorting device. In this way a firmness measurement can be performed on a product in effective manner. It is hereby also possible in relatively simple manner to use the measurement for the sorting as well, if desired in a combination with other measurements.

In an advantageous embodiment according to the invention the detection module comprises a firmness determiner configured to determine the firmness of the product on the basis of properties of the irradiated product surface and/or the scatter surface area. In a currently preferred embodiment the firmness determiner determines the proportion, preferably in the form of the ratio of the irradiated product surface and the scatter surface area of the product, so as to derive the firmness of the product therefrom. It is additionally or alternatively also possible to determine for instance the difference in (absolute) size between the two surface areas. It will further be apparent that it is additionally or alternatively also possible to compare the radius or diameter of the two surface areas (irradiated and scattered), for instance using the ratio/proportion and/or the difference thereof. If desired, it is also possible to determine a mutual relation in different ways, preferably of both said surface areas, such as a function of the area, radius, diameter or other parameter. In addition to or instead of a proportion, such functions/ways of determining mutual relation can relate to, among other things, a quadratic or other equation of for instance the two surface areas. It is noted that when a fixed irradiated surface area is used, it is optionally possible to limit the detection to a scatter surface area, since the irradiated surface area is then deemed constant.

It has been found that a reliable firmness measurement can be obtained using the proportion. By comparing the proportion to a reference or reference line the firmness can be determined, whereby the product can for instance be classified. By making use of the proportion between the surface areas a self-correcting effect occurs for the size or the type of measuring signal, and possible variation in distance of the product to the light source and/or camera device additionally has less impact. The same also applies for possible variation in product dimensions. The reliability of the obtained firmness measurement is increased considerably hereby. In addition, use is preferably made of one or more filters, whereby the effect of the presence of possible distorting elements, such as stickers, stalks, leaves and the like, is minimal.

In an advantageous embodiment according to the invention at least a part of the light sources of the signal device is placed in series.

By placing at least a part of the light sources in series, particularly at least partially in the transport direction of the products, a firmness measurement can be obtained at a plurality of positions distributed over the product surface. The number of light sources is preferably adapted here to the transport speed of the products, such that measuring takes place over more than one revolution of the product. In a currently preferred embodiment a correction occurs for any surfaces which may have been "double" measured.

The number of light sources preferably amounts to at least 6, more preferably at least 10, still more preferably at least 14, and most preferably amounts to at least 16. It has been found that such a number of light sources significantly increases the reliability of the firmness measurement over the whole surface of the product.

In a currently preferred embodiment at least a part of the number of light sources is offset over a distance, substantially in transverse direction to the transport direction of the product. This is preferably done if the number of light sources is sufficient to make more than one revolution of the product available during the measurement. By offsetting the additional light sources over a certain distance additional firmness information is also obtained in width direction of the product. The number of light sources and the specific position of these light sources are preferably adapted to the usual dimensions of the product in question.

In a further preferred embodiment according to the invention the measuring device is further provided with a projection element configured to form into a beam and project the measuring signal onto the product surface.

By providing a projection element the measuring signal, particularly the light signal, can be formed into a beam and projected in effective manner onto a desired location on the product surface. It is hereby possible to increase for instance the measurement accuracy. Such a projection element comprises for instance a lens, mirror and/or other suitable equipment.

In an advantageous embodiment according to the invention the measuring signal comprises a wavelength in the range of 780 nm to 950 nm, preferably 800 nm to 925 nm, and most preferably lies in the range of 870 nm to 890 nm.

It has been found that application of one or more of these wavelengths in the measuring signal, simultaneously or contiguously, results in reliable and fast firmness measurements for diverse products. It has for instance been found that a wavelength of 880 nm results in suitable firmness measurements for kiwis. According to the invention, it is likewise possible in a possible embodiment to configure a number of light sources, such as lasers, to collectively transmit measuring signals, periodically or successively, with at least two wavelengths, for instance 825 and 880 nm. Experiments have shown that this results in a reliable measurement for kiwis. The relevant wavelengths are preferably adapted to the products to be processed.

In an advantageous embodiment according to the invention the measuring device is provided with a second camera device.

The second camera device is for instance provided at the same angle to the light source as the first camera device. The camera device is preferably provided here with a sufficiently large angular aperture in which an image is observable, such that an effective image of the product with the relevant surface areas thereon is observable.

When 14 or 16 light sources are for instance applied in series, a part of which is optionally offset over a distance, 28 or 32 images are obtained. The detection module then preferably determines a median value or percentile value on the basis of these images. The median value or percentile value is for instance determined here per measuring point on the product. It is additionally or alternatively possible if desired to use different values. It is hereby for instance possible to filter out measurements which cause distortion as a result of for instance stickers which may be present or other distortions on the product. It has been found that, depending on the type of product, a correction of the measurements is hereby possible, whereby the reliability of the measurement can be increased still further.

In an advantageous embodiment according to the invention the angle between the signal device and the at least one camera device lies in the range of 0° to 180°, preferably in the range of 10° to 170°, preferably in the range of 20° to 90°, more preferably in the range of 30° to 45°, and most preferably in the range of 35° to 40°. It has been found that said range for the angle between the signal device and the at least one camera device results in an effective measurement wherein a reliable signal, and a reliable image recording, are obtained without for instance reflections having a great deal of impact. It is alternatively or additionally possible to provide the camera device at a minimal angle to or in line with the measuring signal of the signal device. Possible effects of distorting reflections are then counteracted by for instance performing a plurality of measurements and suppressing the distorting measurements using a percentile filter.

In a further advantageous embodiment according to the invention the measuring device comprises a multispectral device configured to carry out a spectral analysis on the product.

By expanding the measuring device according to the invention with a multispectral device it is possible to combine measurements for the purpose of improving the firmness measurements. It is thus for instance possible to determine the location of stickers and other distortions and to omit or at least correct the measurements at such positions. Other parameters can also be determined. Such a multispectral measuring device is for instance described in NL 2017235. It is also possible if desired to apply a temperature correction.

The invention further relates to a sorting system for sorting products, such as fruit and vegetables, comprising:
- a measuring device according to the invention as described above; and
- a sorting device configured to sort the products on the basis of the firmness measured with the measuring device.

Such a sorting system provides similar advantages and effects as described for the measuring device. It is hereby possible to carry out a sorting of products on the basis of firmness. It is also possible if desired to include optional other parameters or quantities in the sorting, for instance by making use of a multispectral device.

The invention further also relates to a method for measuring firmness of products according to claim 12.

In an advantageous embodiment according to the invention the method further comprises of determining properties of the scatter surface area and/or the irradiated product surface in order to determine the firmness of the product. In a currently preferred embodiment the method comprises of determining the proportion, for instance the ratio, of the irradiated product surface and the scatter surface area of the product. It has been found that this proportion between said dimensions provides a reliable indication of firmness.

In a currently preferred embodiment the method further comprises of transmitting a measuring signal with a second wavelength and comparing irradiated product surfaces and/or scatter surface areas with different wavelengths.

It has been found that such signals with different wavelengths can be transmitted individually or can be combined into a single measuring signal. The proportions are hereby similar with the different wavelengths, and it has been found that the firmness measurement can be improved further.

In a possible embodiment according to the invention the method comprises a step of pulsating the signal device. Pulsating makes it possible to alternate the measuring signal with a measuring signal with different wavelength(s) and/or other types of measurement, such as multispectral measurements, without these measurements impeding each other. In the case of a combination with a multispectral measurement it is here possible to place a so-called blocking filter on the spectral camera in order to filter out possible effects of the light source, such as the laser, for the measuring device according to the invention. In this way it is possible to leave the light sources on continuously without this having any significant undesirable effect on the other measurements.

The method preferably further comprises of sorting the products on the basis of the measured firmness. It is here possible if desired to take other parameters or quantities into consideration.

Further advantages, features and details of the invention are further elucidated below on the basis of preferred embodiments thereof, wherein reference is made to the accompanying drawings, in which:
- figures 1A and 1B show views of a measuring device according to the invention;
- figures 2A and 2B show views of the basic operation of the measurement according to the invention in two embodiments;
- figure 3A shows an overview of obtained measuring images of a hard (kiwi) product;
- figure 3B shows views of measurements of a soft (kiwi) product;
- figures 4A and 4B show obtained measurement results; and
- figure 5 shows a view of a sorting system provided with the measuring device of figures 1A and 1B.

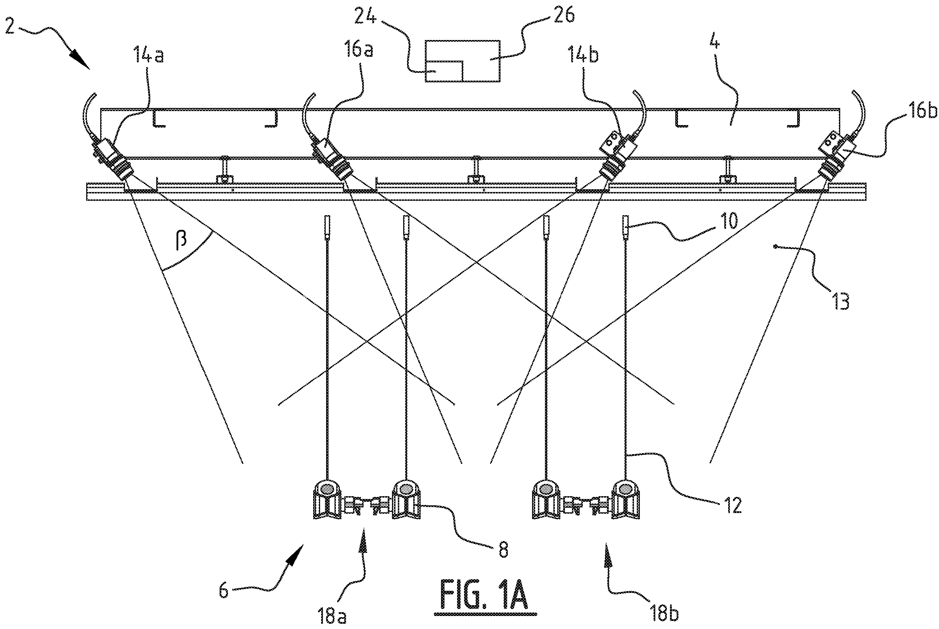

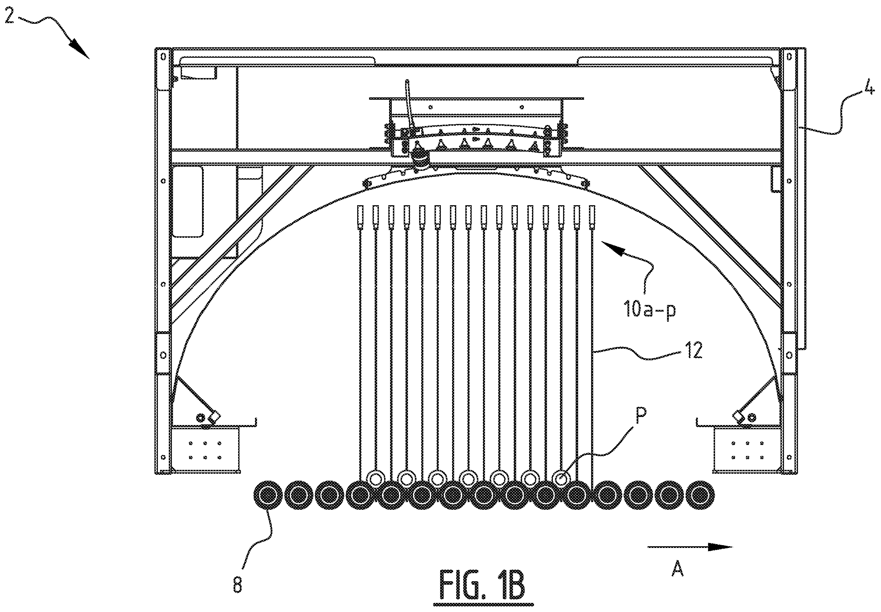

Measuring device 2 (figures 1A and 1B) is provided with frame 4 under which or in which conveyor or transport device 6 is wholly or partially arranged. In the shown embodiment conveyor 6 is provided with a number of so-called diabolos 8 over which product P can be transported in transport direction A. Lasers 10 are provided at a distance above diabolos 8. In the shown embodiment lasers 10a... p are provided in series in transport direction A. When a firmness measurement is performed, measuring signal 12 is transmitted from laser 10 toward conveyor 6, and particularly toward product P. It will be apparent that it is also possible to apply other suitable light sources as alternative to or in addition to lasers 10.

In the shown embodiment a first set of cameras 14a,b is provided, as is a second set of cameras 16a,b for the respective tracks 18a,b. In this way measuring can effectively take place with a double camera 14 on a single track 18. If desired, it is also possible to make use of a plurality of (double) cameras 14, 16 per track 18, or to sample a plurality of tracks 18 with a set of cameras 14, 16.

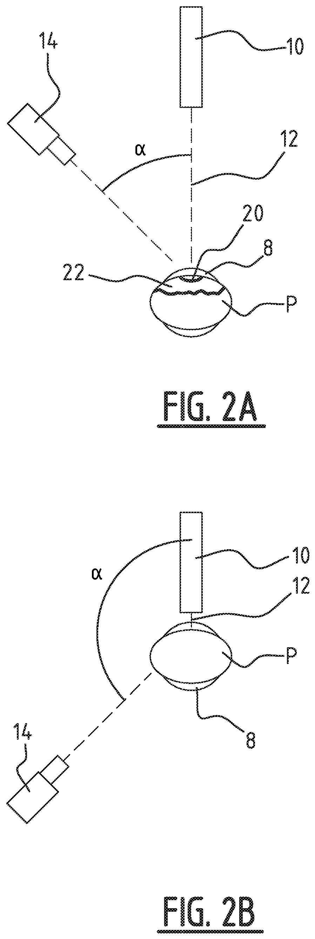

During the firmness measurement, measuring signal 12 is transmitted from laser 10 to the surface of product P. An irradiated surface area or spot 20 (figures 2A, 2B) is here determined with image signal 13 of camera 14, 16 with camera angle β. A scatter surface area or scatter region 22 becomes visible under the surface to camera 14, 16, wherein measuring signal 12 has a significant effect.

Regions 20, 22 are determined using detection module or vision module 24. In the shown embodiment the detection module is shown schematically as part of controller 26, which is configured to control lasers 10 and cameras 14, 16. In the shown embodiment controller 26 also functions as synchronizer.

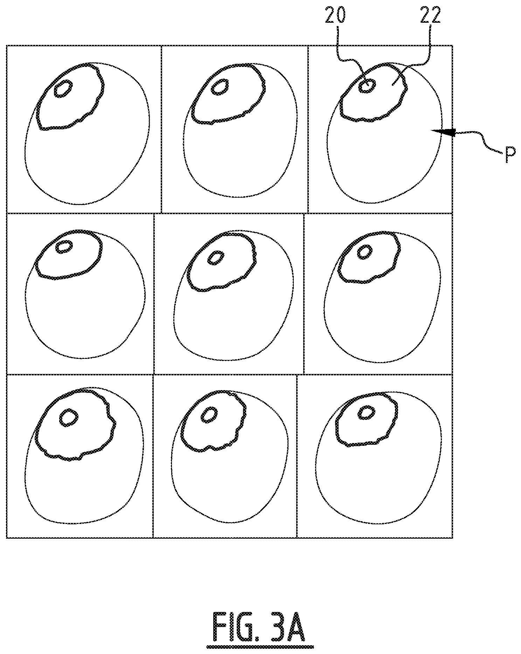

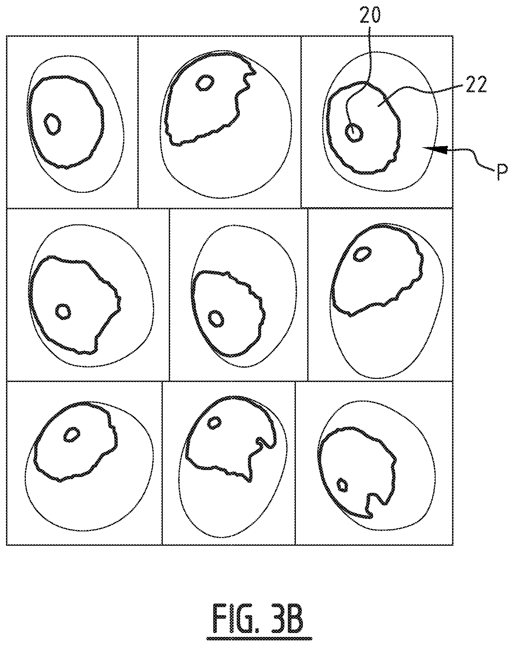

In the shown embodiment camera 14 is provided at an angle α to measuring signal 12. In a possible embodiment α is smaller than 90° (figure 2A) or conversely greater than 90° (figure 2B). Obtained images are analysed using detection module 24, whereby the surface areas of spot 20 and region 22 are determined (figures 3A, 3B). In the case of harder products the scatter region is relatively small relative to the spot region 20. In the case of soft products (figure 3B) on the other hand, the scatter region 22 is much larger in proportion to the spot 20. In this way a measure of the firmness of products P can be determined in effective manner.

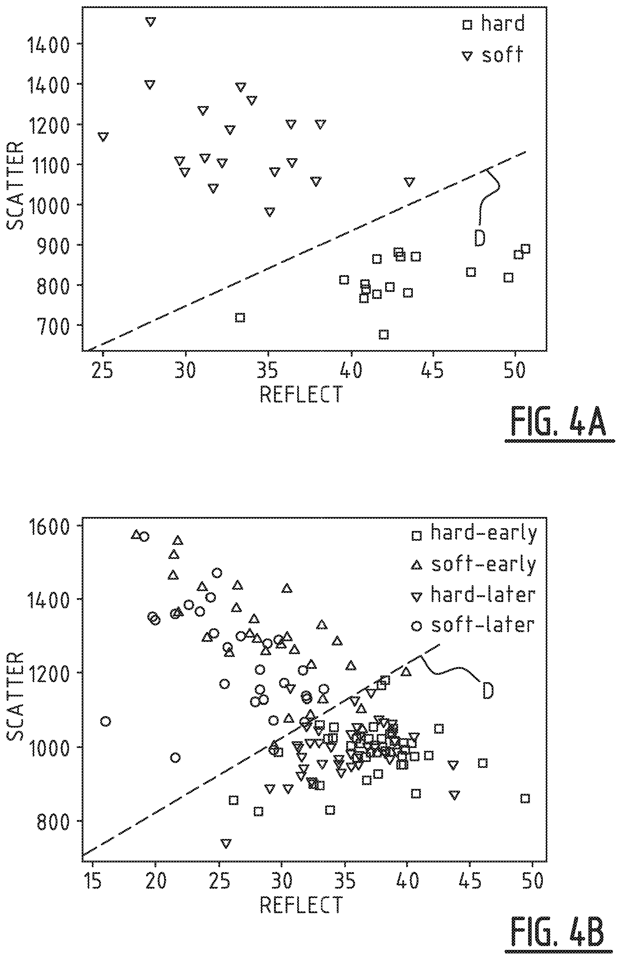

In an experiment forty kiwis are sampled with measuring device 2. The surface areas of spot 20 and scatter region 22 are determined here. In the shown embodiment results are expressed in the number of pixels (figures 4A, 4B) for spot 20 as x-axis and for scatter region 22 as y-axis. A first measurement shows that a distinction between soft and hard kiwis is possible. In the shown results products to the left above detection line D are designated as soft and those on the other side of detection line D are designated as hard. For calibration purposes the products are also sampled offline so that a reliable online measurement can be obtained with measuring device 2 according to the invention.

In a further experiment the products, in the shown representation kiwis, are sampled both early in the morning and late in the afternoon in order to be able to determine the effect of time and/or conditions on the robustness of the measurements. In this experiment it has also been found possible to make a fairly robust division between hard and soft products, irrespective of the time of measurement.

It will be apparent that it is also possible if desired to measure other products, and also to determine a plurality of classes. It is also possible if desired to create a gradation of the distance to detection line D by determining the length of the perpendicular line from this measuring point to the detection line D and to use this as a measure for the actual softness or hardness of the product, and thereby enable for instance a mutual comparison of products within the same class. Additional information about products P can hereby be obtained.

For the purpose of performing a measurement products are placed on a conveyor 6, for instance provided with a number of diabolos 8. While advancing in transport direction A, products P rotate about their own axis such that a different part of the product surface of product P is irradiated at each individual laser 10. By forming at least one image at each of these positions using camera 14, 16 spot 20 and scatter region 22 can be determined for each measurement. Comparison to a threshold and/or detection line D makes it possible to designate a firmness indication in the form of hard, soft or further specified class indicators. The value of the firmness is optionally quantified by determining the distance of the measuring point to the nearby detection line or detection lines D.

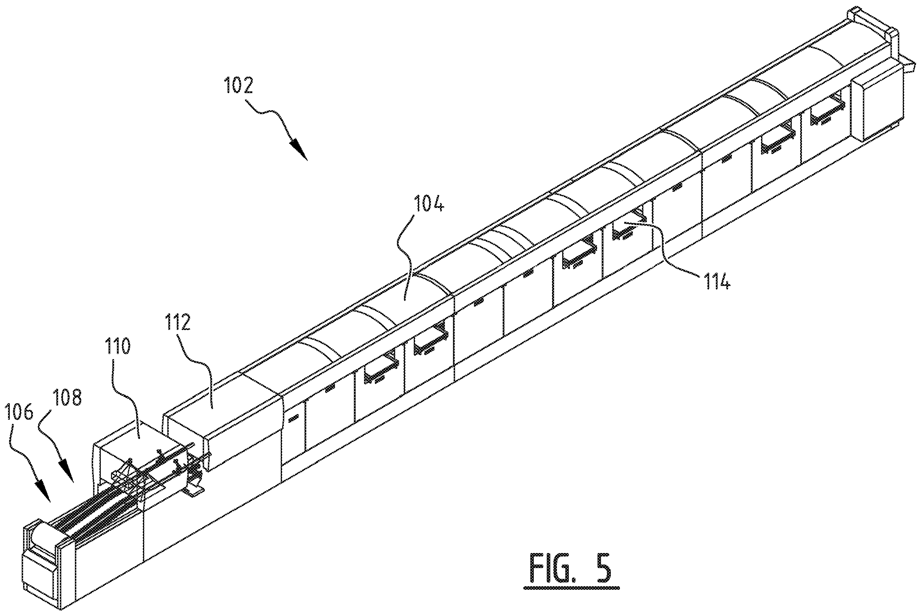

In the shown embodiment sorting system 102 (figure 5) is provided with sorting device 104, feed system 106 and singulator 108. In this embodiment measuring system 110 is provided with, among other things, measuring device 2 and performs one or more measurements on products P from the product flow. After they are measured with measuring system 110, transfer unit 112 places products P in carriers or containers of sorting device 104, whereby products P are transported to sorting outlets 114. It is preferably determined on the basis of the measurements which sorting outlet 114 will form the destination for a specific product P.

The present invention is by no means limited to the above described preferred embodiments thereof. The scope of the invention is defined by the appended claims.

## Claims

1. Measuring device (2) for measuring firmness of products (P), such as fruit and vegetables, comprising:
- a signal device provided with one or more light sources (10) configured to transmit a number of measuring signals (12) to the product while the product is being advanced in a transport direction (A) with a transport device;
- a camera device (14,16) placed at an angle to and/or in line with the signal device and configured to record a product surface (20) irradiated with the measuring signal and a scatter surface area (22) of the measuring signal around the irradiated product surface while the product is being advanced; and
- a detection module (24) connected operatively to the signal device and camera device and configured to determine the firmness of the product on the basis of obtained camera images, wherein the camera images comprise an irradiated product surface and a scatter surface area.

2. Measuring device according to claim 1, wherein the detection module comprises a firmness determiner configured to determine the firmness of the product on the basis of properties of the irradiated product surface and/or the scatter surface area.

3. Measuring device according to claim 2, wherein the firmness determiner is further configured to determine the proportion of the irradiated product surface and the scatter surface area of the product.

4. Measuring device according to claim 1, 2 or 3, wherein at least a part of the light sources of the signal device is placed in series, wherein the number of light sources preferably amounts to at least 6, more preferably at least 10, even more preferably at least 14, and most preferably at least 16, wherein preferably at least a part of the number of light sources is offset over a distance, substantially in transverse direction to the transport direction of the product.

5. Measuring device according to any one of the foregoing claims, wherein the number of light sources comprise a number of lasers (10).

6. Measuring device according to any one of the foregoing claims, further comprising a projection element configured to form into a beam and project the measuring signal onto the product surface.

7. Measuring device according to any one of the foregoing claims, wherein the measuring signal is provided with a wavelength in the range of 780 nm to 950 nm, preferably in the range of 800 nm to 925 nm, and most preferably in the range of 870 nm to 890 nm.

8. Measuring device according to any one of the foregoing claims, further comprising a second camera device (16).

9. Measuring device according to any one of the foregoing claims, wherein the angle between the signal device and the at least one camera device lies in the range of 0° to 180°, preferably in the range of 10° to 170°, more preferably in the range of 20° to 90°, still more preferably in the range of 30° to 45°, and most preferably in the range of 35° to 40°.

10. Measuring device according to any one of the foregoing claims, further comprising a multispectral device configured to carry out a spectral analysis on the product.

11. Sorting system (102) for sorting products (P), such as fruit and vegetables, comprising:
- a measuring device (2) according to any one of the foregoing claims; and
- a sorting device (104) configured to sort the products on the basis of the firmness measured with the measuring device.

12. Method for measuring firmness of products (P), such as fruit and vegetables, comprising the steps of:
- providing a measuring device (2) according to any one of the claims 1-10;
- transmitting a number of measuring signals (12) to the product with the signal device, provided with a number of light sources (10), while the product is being advanced with a transport device (6);
- recording the product surface (20) irradiated with the measuring signal and the scatter surface area of the measuring signal under the product surface using a camera device (14,16) placed at an angle to and/or in line with the signal device while the product is being advanced; and
- determining the firmness of the product on the basis of obtained camera images using a detection module (24) connected operatively to the signal device and camera device, wherein the camera images comprises an irradiated product surface and a scatter surface area.

13. Method according to claim 12, further comprising of determining properties of the irradiated product surface and/or the scatter surface area.

14. Method according to claim 13, further comprising of determining the proportion of the irradiated product surface and the scatter surface area of the product.

15. Method according to claim 13 or 14, further comprising of transmitting a measuring signal with a second wavelength and comparing irradiated product surfaces and/or scatter surface areas at different wavelengths, and/or further comprising the step of pulsating the signal device, and/or further comprising the step of sorting the products on the basis of the firmness measured with the measuring device.

## Patentansprüche

1. Messvorrichtung (2) zum Messen einer Festigkeit von Produkten (P), wie Obst und Gemüse, umfassend:
- eine Signalvorrichtung, die mit einer oder mehreren Lichtquellen (10) versehen ist, die konfiguriert ist, um eine Anzahl von Messsignalen (12) an das Produkt zu übertragen, während das Produkt mit einer Transportvorrichtung in eine Transportrichtung (A) vorwärts bewegt wird;
- eine Kameravorrichtung (14,16), die in einem Winkel zu und/oder in Linie mit der Signalvorrichtung platziert ist und konfiguriert ist, um eine Produktoberfläche (20), die mit dem Messsignal bestrahlt wird, und einen Streuoberflächenbereich (22) des Messsignals um die bestrahlte Produktoberfläche aufzuzeichnen, während das Produkt vorwärts bewegt wird; und
- ein Detektionsmodul (24), das mit der Signalvorrichtung und der Kameravorrichtung wirkverbunden ist und konfiguriert ist, um die Festigkeit des Produkts auf der Basis erhaltener Kamerabilder zu bestimmen, wobei die Kamerabilder eine bestrahlte Produktoberfläche und einen Streuoberflächenbereich umfassen.

2. Messvorrichtung nach Anspruch 1, wobei das Detektionsmodul einen Festigkeitsbestimmer, der konfiguriert ist, um die Festigkeit des Produkts auf der Basis von Eigenschaften der bestrahlten Produktoberfläche und/oder des Streuoberflächenbereichs zu bestimmen, umfasst.

3. Messvorrichtung nach Anspruch 2, wobei der Festigkeitsbestimmer ferner konfiguriert ist, um den Anteil der bestrahlten Produktoberfläche und des Streuoberflächenbereichs des Produkts zu bestimmen.

4. Messvorrichtung nach Anspruch 1, 2 oder 3, wobei mindestens ein Teil der Lichtquellen der Signalvorrichtung in Reihe platziert ist, wobei die Anzahl von Lichtquellen vorzugsweise mindestens 6, mehr bevorzugt mindestens 10, noch mehr bevorzugt mindestens 14 und am meisten bevorzugt mindestens 16, beträgt, wobei vorzugsweise mindestens ein Teil der Anzahl von Lichtquellen über eine Distanz, im Wesentlichen in eine Querrichtung zu der Transportrichtung des Produkts, versetzt ist.

5. Messvorrichtung nach einem der vorstehenden Ansprüche, wobei die Anzahl von Lichtquellen eine Anzahl von Lasern (10) umfasst.

6. Messvorrichtung nach einem der vorstehenden Ansprüche, ferner umfassend ein Projektionselement, das konfiguriert ist, um sich in einen Strahl auszubilden und das Messsignal auf die Produktoberfläche zu projizieren.

7. Messvorrichtung nach einem der vorstehenden Ansprüche, wobei das Messsignal mit einer Wellenlänge in dem Bereich von 780 nm bis 950 nm, vorzugsweise in dem Bereich von 800 nm bis 925 nm und am meisten bevorzugt in dem Bereich von 870 nm bis 890 nm, versehen ist.

8. Messvorrichtung nach einem der vorstehenden Ansprüche, ferner umfassend eine zweite Kameravorrichtung (16).

9. Messvorrichtung nach einem der vorstehenden Ansprüche, wobei der Winkel zwischen der Signalvorrichtung und der mindestens einen Kameravorrichtung in dem Bereich von 0° bis 180°, vorzugsweise in dem Bereich von 10° bis 170°, mehr bevorzugt in dem Bereich von 20° bis 90°, noch mehr bevorzugt in dem Bereich von 30° bis 45° und am meisten bevorzugt in dem Bereich von 35° bis 40°, liegt.

10. Messvorrichtung nach einem der vorstehenden Ansprüche, ferner umfassend eine Multispektralvorrichtung, die konfiguriert ist, um eine Spektralanalyse des Produkts auszuführen.

11. Sortiersystem (102) zum Sortieren von Produkten (P), wie Obst und Gemüse, umfassend:
- eine Messvorrichtung (2) nach einem der vorstehenden Ansprüche; und
- eine Sortiervorrichtung (104), die konfiguriert ist, um die Produkte auf der Basis der Festigkeit, die mit der Messvorrichtung gemessen wird, zu sortieren.

12. Verfahren zum Messen der Festigkeit von Produkten (P), wie Obst und Gemüse, umfassend die Schritte:
- Bereitstellen einer Messvorrichtung (2) nach einem der Ansprüche 1 bis 10;
- Übertragen einer Anzahl von Messsignalen (12) an das Produkt mit der Signalvorrichtung, die mit einer Anzahl von Lichtquellen (10) versehen ist, während das Produkt mit einer Transportvorrichtung (6) vorwärts bewegt wird;
- Aufzeichnen der Produktoberfläche (20), die mit dem Messsignal bestrahlt wird, und des Streuoberflächenbereichs des Messsignals unter der Produktoberfläche unter Verwendung einer Kameravorrichtung (14,16), die in einem Winkel zu und/oder in Linie mit der Signalvorrichtung platziert ist, während das Produkt vorwärts bewegt wird; und
- Bestimmen der Festigkeit des Produkts auf der Basis erhaltener Kamerabilder unter Verwendung eines Detektionsmoduls (24), das mit der Signalvorrichtung und der Kameravorrichtung wirkverbunden ist, wobei die Kamerabilder eine bestrahlte Produktoberfläche und einen Streuoberflächenbereich umfassen.

13. Verfahren nach Anspruch 12, ferner umfassend das Bestimmen von Eigenschaften der bestrahlten Produktoberfläche und/oder des Streuoberflächenbereichs.

14. Verfahren nach Anspruch 13, ferner umfassend das Bestimmen des Anteils der bestrahlten Produktoberfläche und des Streuoberflächenbereichs des Produkts.

15. Verfahren nach Anspruch 13 oder 14, ferner umfassend das Übertragen eines Messsignals mit einer zweiten Wellenlänge und ein Vergleichen bestrahlter Produktoberflächen und/oder von Streuoberflächenbereichen bei unterschiedlichen Wellenlängen, und/oder ferner umfassend den Schritt eines Pulsierens der Signalvorrichtung, und/oder ferner umfassend den Schritt des Sortierens der Produkte auf der Basis der Festigkeit, die mit der Messvorrichtung gemessen wird.

## Revendications

1. Dispositif de mesure (2) permettant de mesurer la fermeté de produits (P), tels que des fruits et légumes, comprenant :
- un dispositif de signalisation doté d'une ou plusieurs sources lumineuses (10) configuré pour transmettre un certain nombre de signaux de mesure (12) au produit pendant que le produit est avancé dans une direction de transport (A) à l'aide d'un dispositif de transport ;
- un dispositif de caméra (14, 16) placé à un angle et/ou de façon alignée avec le dispositif de signalisation et configuré pour enregistrer une surface de produit (20) exposée au signal de mesure et une zone de surface de diffusion (22) du signal de mesure autour de la surface de produit exposée pendant que le produit est avancé ; et
- un module de détection (24) connecté fonctionnellement au dispositif de signal et au dispositif de caméra et configuré pour déterminer la fermeté du produit sur la base d'images de caméra obtenues, dans lequel les images de caméra comprennent une surface de produit exposée et une zone de surface de diffusion.

2. Dispositif de mesure selon la revendication 1, dans lequel le module de détection comprend un dispositif de détermination de fermeté configuré pour déterminer la fermeté du produit sur la base de propriétés de la surface de produit exposée et/ou de la zone de surface de diffusion.

3. Dispositif de mesure selon la revendication 2, dans lequel le dispositif de détermination de fermeté est en outre configuré pour déterminer la proportion de la surface de produit exposée et de la zone de surface de diffusion du produit.

4. Dispositif de mesure selon la revendication 1, 2 ou 3, dans lequel au moins une partie des sources lumineuses du dispositif de signalisation est placée en série, dans lequel le nombre de sources lumineuses s'élève de préférence à au moins 6, plus préférablement à au moins 10, encore plus préférablement à au moins 14, et le plus préférablement à au moins 16, dans lequel de préférence au moins une partie du nombre de sources lumineuses est décalée sur une distance, sensiblement dans la direction transversale par rapport à la direction de transport du produit.

5. Dispositif de mesure selon l'une quelconque des revendications qui précèdent, dans lequel le nombre de sources lumineuses comprend un certain nombre de lasers (10).

6. Dispositif de mesure selon l'une quelconque des revendications qui précèdent, comprenant en outre un élément de projection configuré pour former un faisceau et projeter le signal de mesure sur la surface de produit.

7. Dispositif de mesure selon l'une quelconque des revendications qui précèdent, dans lequel le signal de mesure est doté d'une longueur d'onde dans la plage de 780 nm à 950 nm, de préférence dans la plage de 800 nm à 925 nm, et le plus préférablement dans la plage de 870 nm à 890 nm.

8. Dispositif de mesure selon l'une quelconque des revendications qui précèdent, comprenant en outre un deuxième dispositif de caméra (16).

9. Dispositif de mesure selon l'une quelconque des revendications qui précèdent, dans lequel l'angle entre le dispositif de signalisation et l'au moins un dispositif de caméra au moins se trouve dans la plage de 0 ° à 180 °, de préférence dans la plage de 10 ° à 170 °, plus préférablement dans la plage de 20 ° à 90 °, plus préférablement encore dans la plage de 30 ° à 45 °, et le plus préférablement dans la plage de 35 ° à 40 °.

10. Dispositif de mesure selon l'une quelconque des revendications qui précèdent, comprenant en outre un dispositif multispectral configuré pour effectuer une analyse spectrale sur le produit.

11. Système de tri (102) permettant de trier des produits (P), tels que des fruits et légumes, comprenant :
- un dispositif de mesure (2) selon l'une quelconque des revendications qui précèdent ; et
- un dispositif de tri (104) configuré pour trier les produits sur la base de la fermeté mesurée à l'aide du dispositif de mesure.

12. Procédé permettant de mesurer la fermeté de produits (P), tels que des fruits et légumes, comprenant les étapes consistant à :
- fournir un dispositif de mesure (2) selon l'une quelconque des revendications 1 à 10 ;
- transmettre un certain nombre de signaux de mesure (12) au produit à l'aide du dispositif de signalisation, doté d'un certain nombre de sources lumineuses (10), pendant que le produit est avancé à l'aide d'un dispositif de transport (6) ;
- enregistrer la surface de produit (20) exposée au signal de mesure et la zone de surface de diffusion du signal de mesure sous la surface de produit à l'aide d'un dispositif de caméra (14, 16) placé à un angle et/ou de façon alignée avec le dispositif de signalisation pendant que le produit est avancé ; et
- déterminer la fermeté du produit sur la base d'images de caméra obtenues à l'aide d'un module de détection (24) connecté fonctionnellement au dispositif de signalisation et au dispositif de caméra, dans lequel les images de caméra comprennent une surface de produit exposée et une zone de surface de diffusion.

13. Procédé selon la revendication 12, comprenant en outre la détermination de propriétés de la surface de produit exposée et/ou de la zone de surface de diffusion.

14. Procédé selon la revendication 13, comprenant en outre la détermination de la proportion de la surface de produit exposée et de la zone de surface de diffusion du produit.

15. Procédé selon la revendication 13 ou 14, comprenant en outre la transmission d'un signal de mesure doté d'une deuxième longueur d'onde et la comparaison des surfaces de produits exposées et/ou des zones de surface de diffusion à différentes longueurs d'onde, et/ou comprenant en outre l'étape consistant à pulser le dispositif de signalisation, et/ou comprenant en outre l'étape consistant à trier les produits sur la base de la fermeté mesurée à l'aide du dispositif de mesure.
